# EUROPEAN PATENT SPECIFICATION

(11) **EP 1 030 618 B1**
(45) Date of publication and mention of the grant of the patent: **21.05.2003**
(21) Application number: 98954701.3
(22) Date of filing: 16.11.1998
(51) Int. Cl.: A61B 19/00, A61B 17/34

(54) **SURGICAL HAND ACCESS DEVICE**
CHIRURGISCHE ZUGANGSVORRICHTUNG FÜR DIE HAND
DISPOSITIF D'ACCES CHIRURGICAL MANUEL

(30) Priority: 14.11.1997 IE 970810
(43) Date of publication of application: 30.08.2000
(73) Proprietor: GAYA LIMITED, Dublin 2 (IE)
(72) Inventor: BONADIO, Frank, Bray County Wicklow (IE)
(74) Representative: McCarthy, Denis Alexis
(86) International application number: IE9800095
(87) International publication number: WO99025268

(56) References cited:
- WO-A-95/07056
- WO-A-95/22289
- US-A- 5 366 478
- US-A- 5 514 133
- US-A- 5 522 791

## Description

The present invention relates to a surgical hand access device, in particular to improvements to a surgical hand access device for minimally invasive surgery.

In minimally invasive surgery gas is generally introduced into a patient's peritoneal cavity to cause pneumoperitoneum. Hand assisted laproscopic surgery (HALS) is a refined form of minimally invasive surgery where a small incision is made in the patient's abdomen and a hand access device is inserted into the incision. Using the hand access device a surgeon can insert his hand into the patient's abdomen and can perform many procedures that cannot be accomplished by standard minimally invasive techniques.

One particularly effective hand access device is that disclosed in our European Patent Specification No. 0 744 922. That device comprises a sleeve having an entry opening located at a proximal end of the sleeve and an exit opening located at a distal end for insertion into an incision made in a patient's body. The sleeve at the exit opening is collapsible by gas pressure within the abdominal cavity of the patient at or adjacent the distal edges of the sleeve. A taut valve, is also provided at the distal edges of the sleeve to reinforce the seal and to anchor the device on the patients body when the inflatable chambers of the device outside the patient's body are inflated by gas pressure. This device is a very effective and simple hand access device.

The present invention provides a hand access port device for use in minimally invasive surgery comprising a flexible tube having an entry opening at a proximal end and an exit opening at a distal end, exit opening sealing means which in use are effected by gas pressure within the abdominal cavity of a patient and sealing means for sealing the entry opening while allowing access for a surgeon's hand or a surgical instrument, an anchoring element, separate from and not attached to the exit opening sealing means, is provided to anchor the device against the abdominal wall of the patient's body cavity, characterised in that the flexible tube comprises an outer sleeve and an inner sleeve which are joined only at the proximal end of the device with a flange being provided at a distal end of the outer sleeve, the flange being mountable on the patient's abdomen around the site of an incision.

Preferably, the anchoring element is a deformable ring which may be inserted through the incision in the patient's body and reverts to its original shape within the patient's body to anchor the device.

Conveniently, an apron is attached to the outer wall of the inner sleeve with the anchoring element being located at the distal end of the apron element.

Preferably, the apron is adapted to be used as a wound protector whereby the outer wall of the apron contacts the surfaces of the incision.

Advantageously, the exit sealing means comprises a cuff valve and the cuff valve comprises a pair of semi rigid strips, each strip being housed in one of a pair of opposed pockets formed at the distal end of the inner sleeve.

Conveniently, the distal end of the outer sleeve is provided with an inflatable toroidal section for contact with the patient's skin.

Advantageously, a third sleeve element is provided within the inner sleeve to act as an intermediate valve about a surgeon's hand between the entry sealing means and the exit sealing means.

Additionally, the anchor element includes a ring element mountable externally of the patient's body.

The device is generally made from flexible, impermeable, biocompatible medically sterile, plastics/polymeric materials.

The invention will hereinafter be more particularly described with reference to the accompany drawings which show by way of example only, a number of embodiments according to the invention. In the drawings: -
Figure 1 is a side view of a first embodiment of surgical hand access device according to the invention;
Figure 2 is a side view of a second embodiment of surgical hand access device according to the invention, having a longer inner sleeve than the first embodiment;

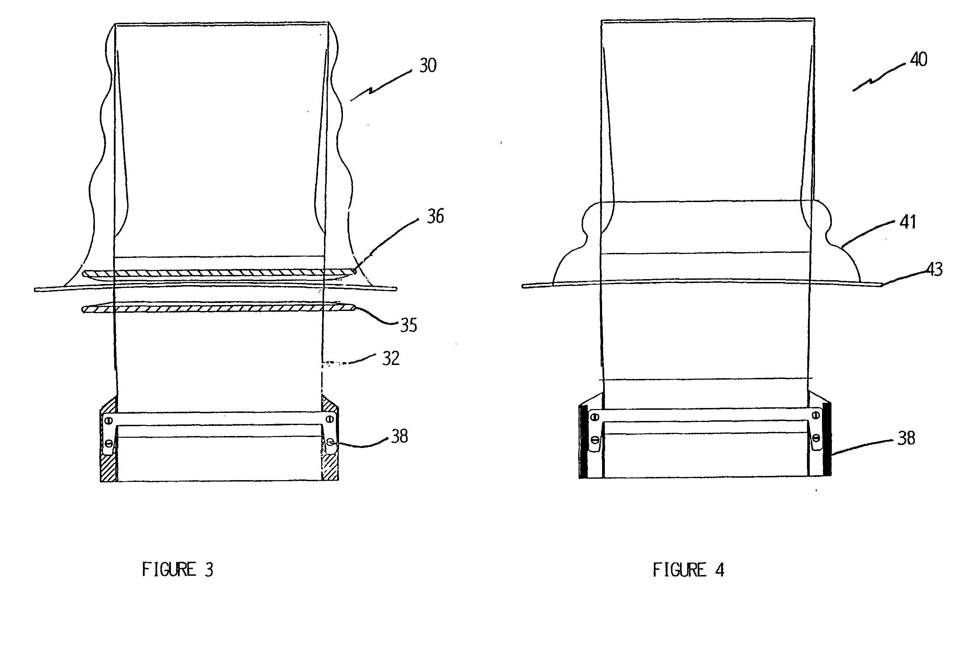

Figures 3 and 4 are side views respectively of third and fourth embodiments of a surgical hand access device;

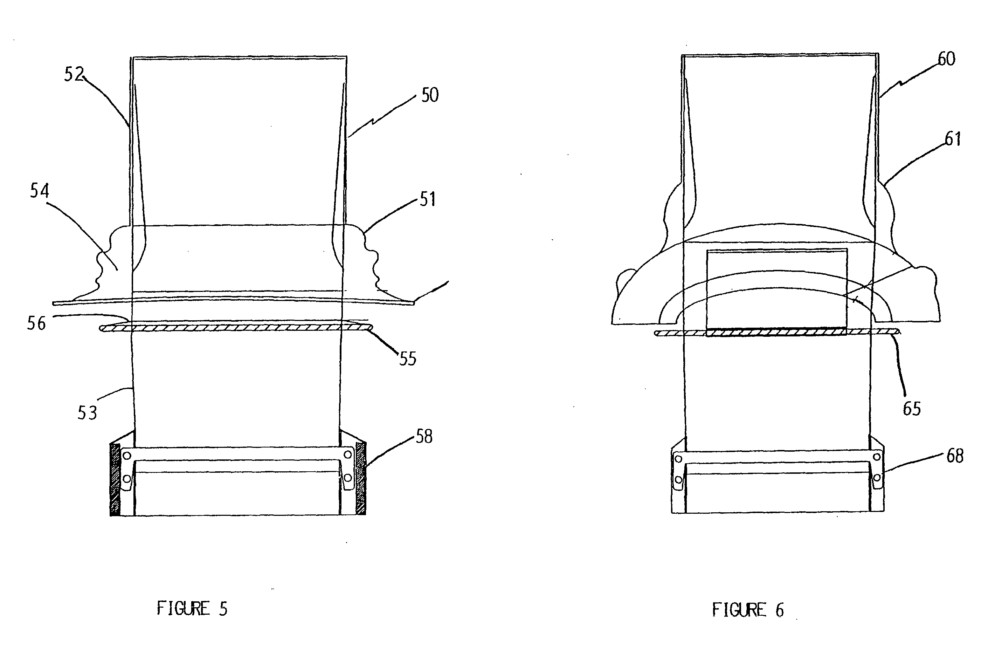

Figures 5 and 6 are side views respectively of fifth and sixth embodiments of a hand access device;
Figure 7 is a side view of a seventh embodiment of a hand access device and Figure 7a is a side view of a modification of the seventh embodiment;

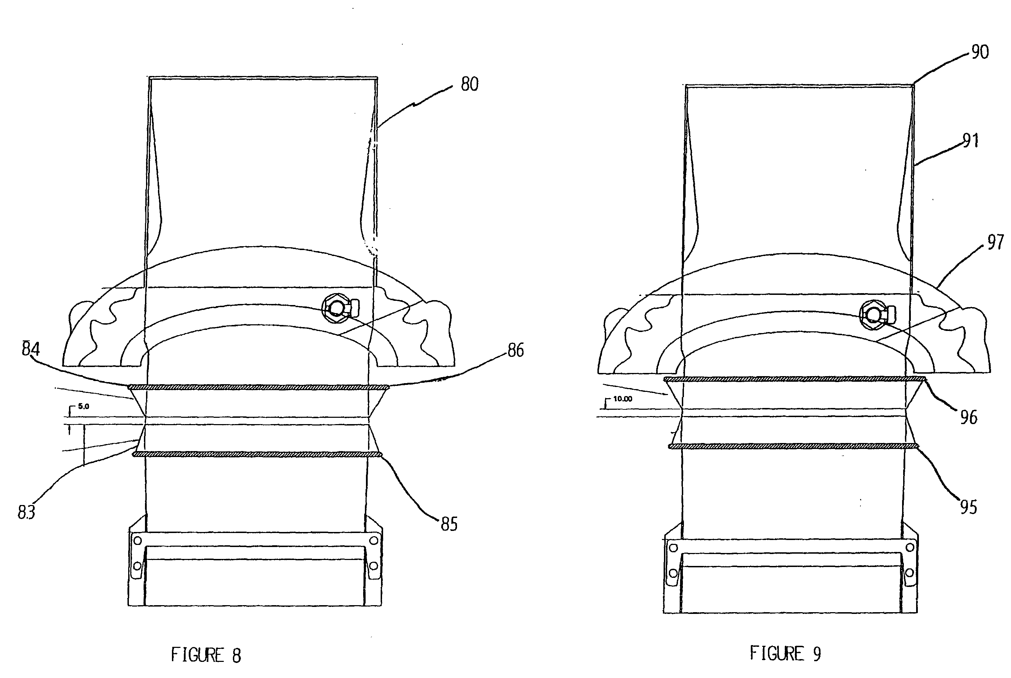

Figures 8 and 9 are side views respectively of an eighth and ninth embodiment of a hand access device;

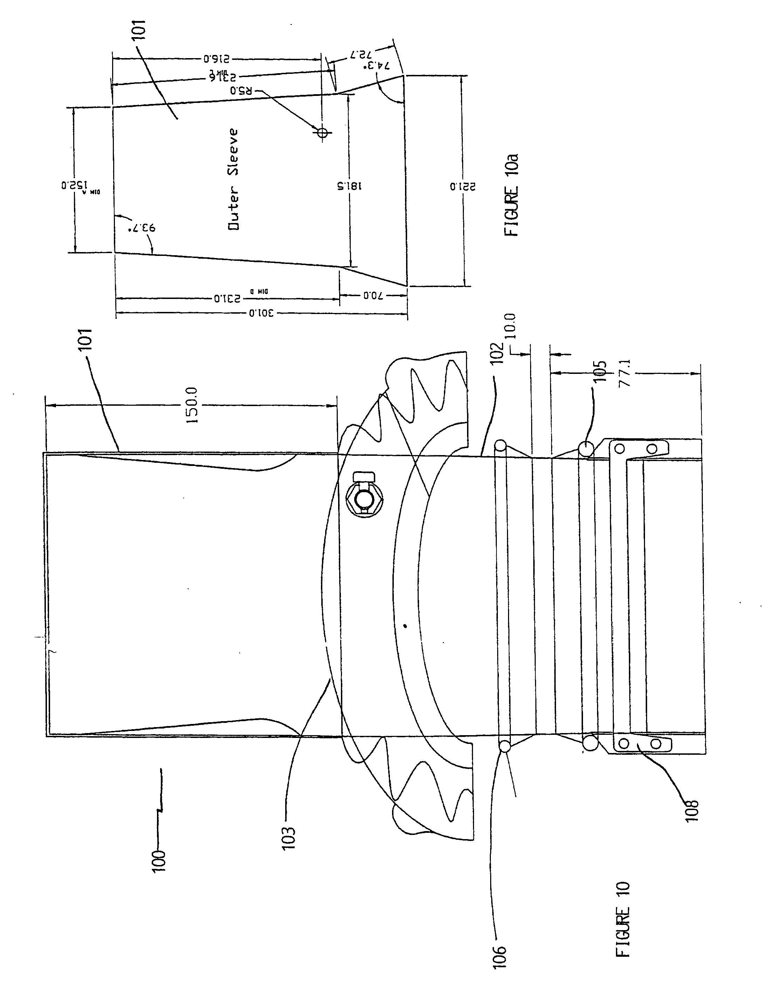

Figure 10 is a side view of a tenth embodiment of a hand access device according to the invention and Figure 10a is a side view of an outer sleeve.
Figure 11 and 11a are a side view respectively of an eleventh embodiment of hand access device according to the invention and the outer sleeve;
Figure 12 is a perspective view of the eleventh embodiment in position on a patient's abdominal wall;
Figure 13 is a perspective view of one end of a cuff valve which can be used in all the above embodiments;
Figure 14 is a perspective view of a hinge and Figure 14a is a perspective view of the hinge applied to the cuff;
Figure 15 is a perspective view of an alternative hinge and Figure 15a is a perspective view of the alternative hinge applied to the cuff.

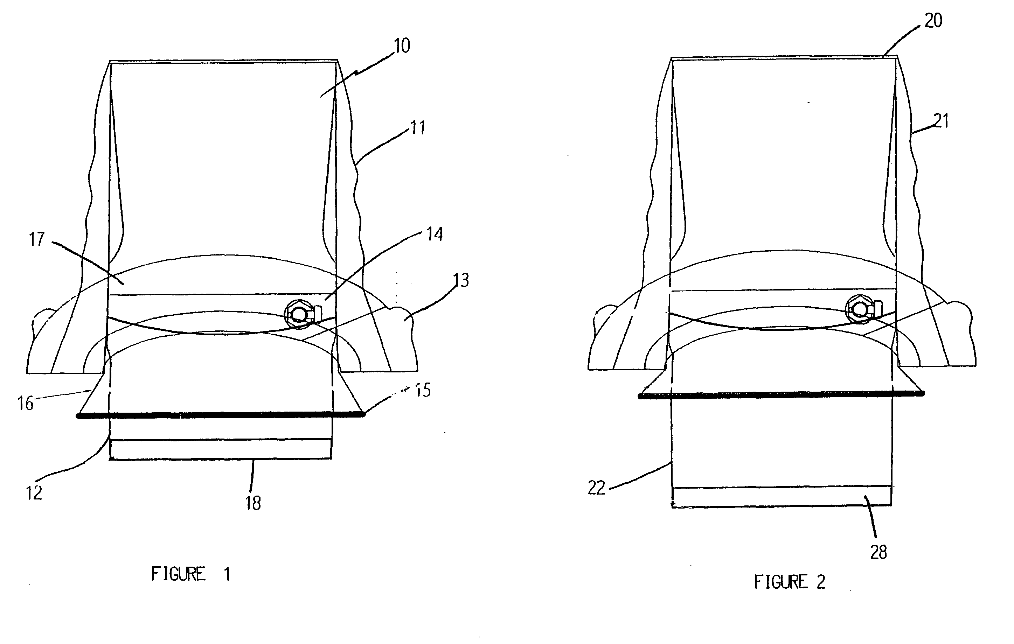

Referring to the drawings, and initially to Figure 1 the hand access device 10 comprises an outer sleeve 11 and an inner sleeve 12, a flange 13, an insulflation valve 14. It is operated in the same manner as our earlier device as described in the above mentioned European patents specification. The device 10 however is fitted with an internal ring 15 which is attached with an apron 16 to the inner sleeve 12 adjacent to the flange 13 below a feather valve 17. The device has no side welds or cross welds and accordingly as the device is inflated with gas the outer sleeve 11 expands and extends upwards and outwards drawing the inner sleeve 12, the attached apron 16 and flexible internal ring 15 upwards. The effect of the upwards movement is to cause the internal ring 15 to act as an anchor below the abdominal wall and for the further purpose of the apron 16 acting as an additional protective barrier between the incision site and the tissue being operated on by the surgeon. Additionally, the drawing up of the apron 16 and internal ring 15 acts to retract the incision site helping to create a wider incision lumen through which a surgeon can insert a hand and forearm. A cuff valve 18 is positioned below the ring 15 and comprises two strips of 1 mm VIVAK 15 mm wide enclosed in a SARANEX pocket at the distal end of the inner sleeve 12. (VIVAK and SARANEX are registered trade marks).

The second embodiment 20 is generally similar to the first embodiment 10 except that the inner sleeve 22 is longer so as to better accommodate the distance from the fingertips to the forearm to allow a seal around the forearm before breaking the seal at the distal valve.

The third embodiment device 30 comprises two rings, an internal ring 35 and an external ring 36. A taut valve 38 is applied at the distal end of the inner sleeve 32.

The fourth embodiment device 40 has a reduced outer sleeve 41 and flame 43. A reinforced taut valve 48 is provided at the distal end of the inner sleeve 42.

The fifth embodiment 50 has a reduced outer sleeve 51 and an internal ring 55. The outer sleeve is reduced in height by shortening the length of the side welds 52 that connect the inner sleeve 53 to the outer sleeve 51. There are no cross welds connecting the inner sleeve 53 and outer sleeve 51. The effect of this is to allow the outer sleeve 51 to move to a greater extent up and down relative to the inner sleeve 53.

In addition the outer sleeve 51 is designed to have extra material at the lower section. The extra material is connected to a flange according to the invention or other conformable flexible material 57. The inclusion of the extra material at the lower section of the outer sleeve allows the lower section to inflate to a greater extent than the upper section. As gas is induced into the device causing the outer sleeve to inflate, the inner sleeve 53 with the apron 56 and internal ring 55 attached is drawn up. The ability of the outer sleeve 51 to draw the inner sleeve 53 up is limited by the internal ring 55 and this limitation causes a ballooning effect of the extra outer sleeve 51 material. The ballooning effect has the result, the external flange 57 and inflated toroid 54 combined with the apron 56 and ring 55 anchored internally acts to create a very effective seal around the incision to the gas and further acts as an aseptic seal to the external air. The inner sleeve 53 acts as an integral wound protector.

The taut valve 58 has struts inside the wings 59. The inner sleeve 52 has side welds which prevent inversion of the inner sleeve. The embodiment 60 has a larger outer sleeve 61, an internal ring 65 and a taut valve 68.

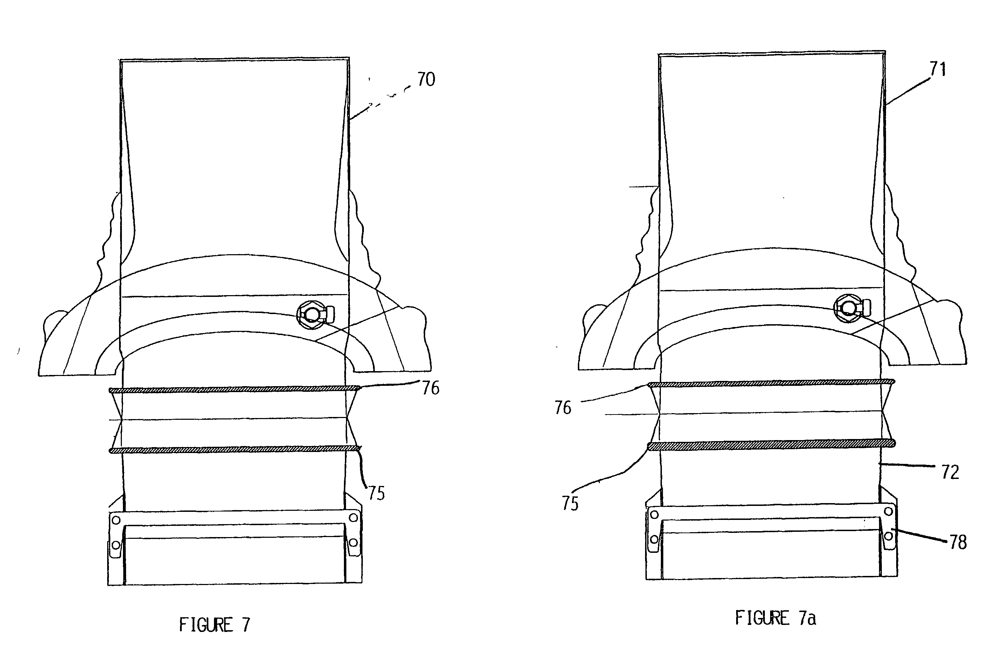

The embodiment 70 has an internal ring 75 and an external ring 76. In Figure 7a the cross section of the internal ring is 8 mm as opposed to 6 mm. The internal ring restricts movement of the taut valve 78 and therefore makes it much easier to pass the hand fully through the valve as far as the forearm.

The embodiment 80 has two rings 85 and 86 and flat weld drapes 83 and 84 as has the embodiment 90 with two rings 95 and 96. The external ring 96 is closer to the flange 97. The anchor rings 95, 96 give very good results with the internal ring taking up most of tenting force. The flexible outer sleeve 91 allows movement of the surgeon's arm without putting strain on any part of the device.

In the tenth embodiment 100, the length of inner sleeve 102 has been increased by 50 mm to allow the taut valve 108 to come up against the internal ring 105. Both of the rings 105 and 106 are made from polyurethane. On inflation of the device there is no gas loss and the tenting force acts on the internal ring. The outer sleeve 101 is very flexible but side welds prevent it from inverting at its proximal end. The inner 102 holds quite taut between its proximal end and the internal ring. A feather valve 103 is used to form a seal about a surgeon's arm.

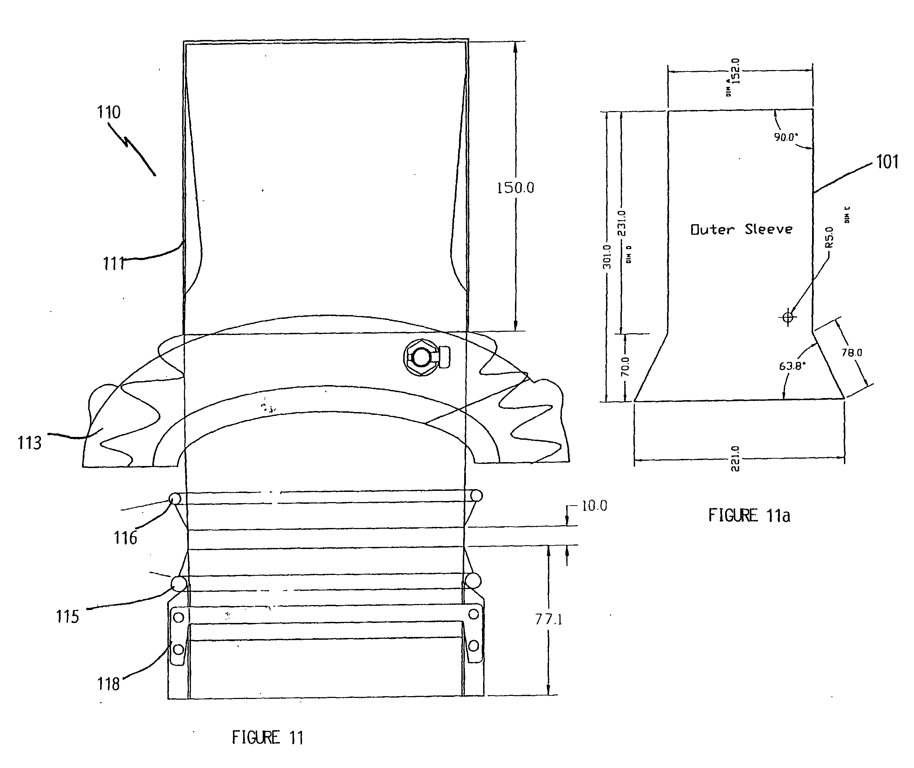

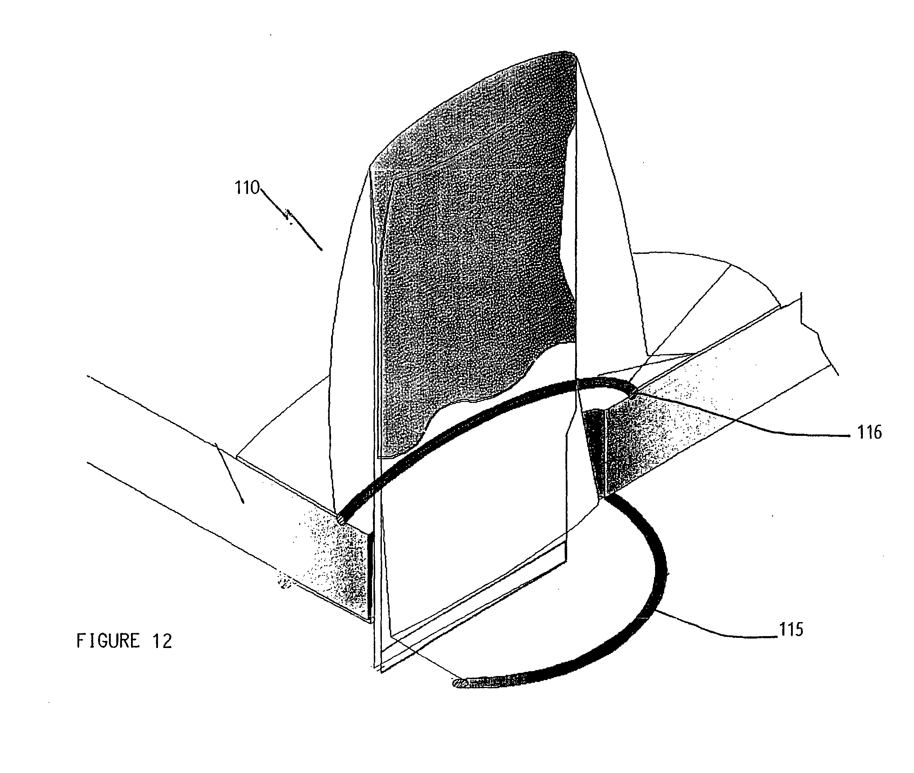

The eleventh embodiment 110 is similar to embodiment 100 except the outer sleeve 111 is configured in a straight line. The sleeve 110 is shown in Figures 11 and 12. The external ring when in position on a patient and the internal ring 115 downloads the force from the flange 113 and protects the taut valve from deflating.

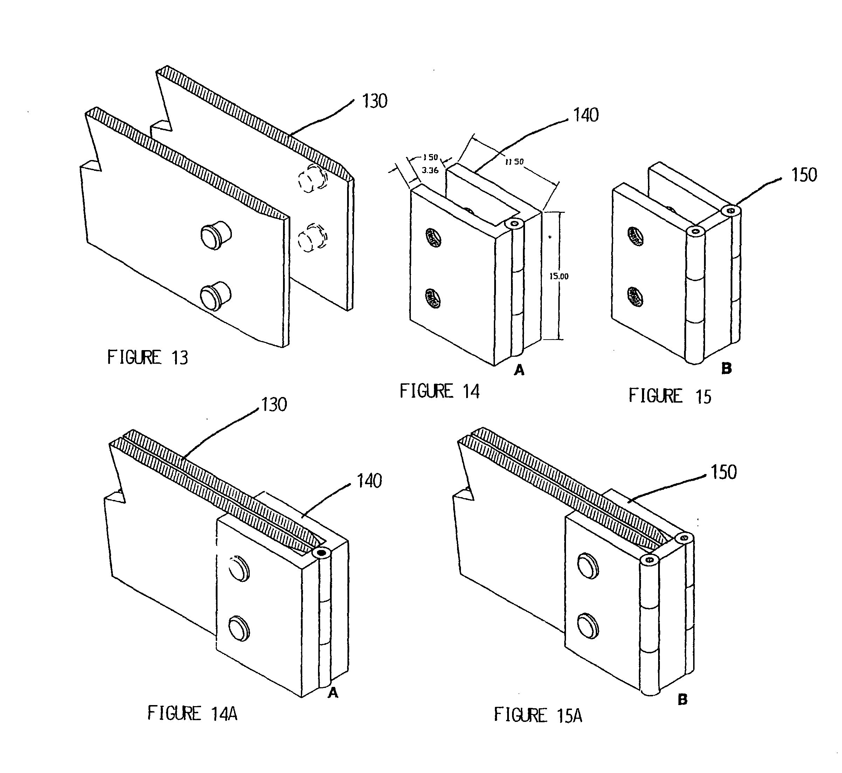

Modifications to the taut valve 130 are shown in Figures 13 to 15a with the use of hinges 140 and 150.

It will of course be understood that the invention is not limited to the specific details described herein, which are given by way of example only, and that various modifications and alterations are possible within the scope of the invention as defined in the appended claims.

## Claims

1. A hand access port device (10) for use in minimally invasive surgery comprising a flexible tube (11,12) having an entry opening at a proximal end and an exit opening at a distal end, exit opening sealing means which in use are effected by gas pressure within the abdominal cavity of a patient and sealing means for sealing the entry opening while allowing access for a surgeon's hand or a surgical instrument, an anchoring element, separate from and not attached to the exit opening sealing means, is provided to anchor the device (10) against the abdominal wall of the patient's body cavity, the flexible tube comprising an outer sleeve (11) and an inner sleeve (12), **characterised in that** the inner and outer sleeves are joined only at the proximal end of the device with a flange (13) being provided at a distal end of the outer sleeve (11), the flange (13) being mountable on the patient's abdomen around the site of an incision.

2. A hand access port device (10) as claimed in Claim 1, in which the anchoring element is a deformable ring (15) which may be inserted through the incision in the patient's body and reverts to its original shape within the patient's body to anchor the device.

3. A hand access port device (10) as claimed in Claim 2, in which an apron (16) is attached to the outer wall of the inner sleeve (12) with the anchoring element being located at the distal end of the apron element (16).

4. A hand access port device (10) as claimed in Claim 3, in which the apron (16) is adapted to be used as a wound protector whereby the outer wall of the apron contacts the surfaces of the incision.

5. A hand access port device (10) as claimed in Claim 2, in which the distal end of the outer sleeve (11) is provided with an inflatable toroidal section (54) for contact with the patient's skin.

6. A hand access device (10) as claimed in any one of the preceding claims, in which the exit sealing means comprises a cuff valve (18).

7. A hand access device (10) as claimed in Claim 6, in which the cuff valve (18) comprises a pair of semi rigid strips, each strip being housed in one of a pair of opposed pockets formed at the distal end of the inner sleeve (12).

8. A hand access port device (10) as claimed in Claim 2, in which a third sleeve element is provided within the inner sleeve to act as an intermediate valve about a surgeon's hand between the entry sealing means and the exit sealing means.

9. A hand access port device (10) as claimed in any one of the preceding claims, in which the anchor element includes a ring element mountable externally of the patient's body.

10. A hand access port device (10) as claimed in any one of the preceding claims in which the device is made from flexible, impermeable, biocompatible medically sterile, plastics/polymeric materials.

## Patentansprüche

1. Handzugrifföffnungsvorrichtung (10) zur Verwendung in der minimal invasiven Chirurgie mit einem flexiblen Schlauch (11, 12), der eine Eintrittsöffnung am proximaien Ende und eine Austrittsöffnung am distalen Ende aufweist, Austrittsöffnungsdichteinrichtungen, die beim Gebrauch durch einen Gasdruck in der Bauchhöhle eines Patienten wirksam werden, und Dichteinrichtungen zum Abdichten der Eintrittsöffnung, wobei Zugang für die Hand eines Chirurgen oder ein chirurgisches Instrument gelassen wird, wobei ein Verankerungselement bereitgestellt ist, das von der Austrittsöffnungsdichteinrichtung getrennt und nicht an dieser angebracht ist, um die Vorrichtung (10) an der Bauchwand der Körperhöhle des Patienten zu verankern, wobei der flexible Schlauch eine äußere Hülle (11) und eine innere Hülle (12) umfasst,
**dadurch gekennzeichnet, dass** die innere und äußere Hülle nur am proximalen Ende der Vorrichtung mit einem Flansch (13) verbunden sind, der an einem distalen Ende der äußeren Hülle (11) bereitgestellt ist, wobei der Flansch (13) am Unterleib des Patienten um die Einschnittstelle befestigbar ist.

2. Handzugrifföffnungsvorrichtung (10) nach Anspruch 1, bei der das Verankerungselement ein verformbarer Ring (15) ist, der durch den Einschnitt im Körper des Patienten eingeführt werden kann und im Körper des Patienten zu seiner ursprünglichen Form zurückkehrt, um die Vorrichtung zu verankern.

3. Handzugrifföffnungsvorrichtung (10) nach Anspruch 2,
bei der an der Außenwand der inneren Hülle (12) eine Schürze (16) angebracht ist, wobei sich das Verankerungselement am distalen Ende des Schürzenelements (16) befindet.

4. Handzugrifföffnungsvorrichtung (10) nach Anspruch 3, bei der die Schürze (16) zur Verwendung als Wundschutz ausgelegt ist, wobei die Außenwand der Schürze die Oberflächen des Einschnitts berührt.

5. Handzugrifföffnungsvorrichtung (10) nach Anspruch 2, bei der das distale Ende der äußeren Hülle (11) mit einem aufblähbaren ringförmigen Abschnitt (54) zum Kontakt mit der Haut des Patienten versehen ist.

6. Handzugriffsvorrichtung (10) nach einem der vorhergehenden Ansprüche, bei der die Austrittsdichteinrichtung ein Schieberventil (18) umfasst.

7. Handzugriffsvorrichtung (10) nach Anspruch 6, bei der das Schieberventil (18) ein Paar halbstarre Streifen umfasst, wobei jeder Streifen in einem Paar einander gegenüberliegender Taschen aufgenommen ist, die am distalen Ende der inneren Hülle (12) ausgeformt sind.

8. Handzugrifföffnungsvorrichtung (10) nach Anspruch 2, bei der ein drittes Hüllenelement in der inneren Hülle bereitgestellt ist, um als Zwischenventil um die Hand eines Chirurgen zwischen der Eintrittsdichteinrichtung der Austrittsdichteinrichtung zu wirken.

9. Handzugrifföffnungsvorrichtung (10) nach einem der vorhergehenden Ansprüche, bei dem das Verankerungselement ein Ringelement umfasst, das außerhalb des Körpers des Patienten befestigbar ist.

10. Handzugrifföffnungsvorrichtung (10) nach einem der vorhergehenden Ansprüche, bei der die Vorrichtung aus flexiblen, undurchlässigen, biokompatiblen, medizinisch sterilen Kunststoff-/Polymermaterialien gefertigt ist.

## Revendications

1. Dispositif (10) à orifice d'accès manuel destiné à être utilisé en chirurgie très peu traumatisante, comprenant un tube souple (11, 12) ayant une ouverture d'entrée à une extrémité proximale et une ouverture de sortie à une extrémité distale, un dispositif d'étanchéité d'ouverture de sortie qui, pendant l'utilisation, est sous l'action de la pression d'un gaz dans la cavité abdominale d'un patient, et un dispositif d'étanchéité destiné à fermer de manière étanche l'ouverture d'entrée tout en permettant l'accès par la main d'un chirurgien ou par un instrument chirurgical, un élément d'ancrage séparé du dispositif d'étanchéité de l'ouverture de sortie et qui n'est pas fixé à ce dispositif, destiné à fixer le dispositif (10) contre la paroi abdominale de la cavité du corps du patient, le tube souple comprenant un manchon externe (11) et un manchon interne (12), **caractérisé en ce que** les manchons interne et externe sont raccordés uniquement à l'extrémité proximale du dispositif par un flasque (13) placé à une extrémité distale du manchon externe (11), le flasque (13) étant destiné à être monté sur l'abdomen du patient autour du site d'une incision.

2. Dispositif (10) à orifice d'accès manuel selon la revendication 1, dans lequel l'élément d'ancrage est un anneau déformable (15) qui peut être inséré par l'incision dans le corps du patient et reprend sa forme originale dans le corps du patient pour l'ancrage du dispositif.

3. Dispositif (10) à orifice d'accès manuel selon la revendication 2, dans lequel un tablier (16) est fixé à la paroi externe du manchon interne (12), l'élément d'ancrage étant disposé à l'extrémité distale de l'élément de tablier (16).

4. Dispositif (10) à orifice d'accès manuel selon la revendication 3, dans lequel le tablier (16) est destiné à être utilisé comme organe protecteur contre les blessures, si bien que la paroi externe du tablier est au contact des surfaces de l'incision.

5. Dispositif (10) à orifice d'accès manuel selon la revendication 2, dans lequel l'extrémité distale du manchon externe (11) possède un tronçon toroïdal gonflable (54) destiné à être au contact de la peau d'un patient.

6. Dispositif (10) d'accès manuel selon l'une quelconque des revendications précédentes, dans lequel le dispositif d'étanchéité de sortie comporte une soupape à manchette (18).

7. Dispositif (10) d'accès manuel selon la revendication 6, dans lequel la soupape à manchette (18) comprend deux bandes semi-rigides, chaque bande étant logée dans l'une de deux poches opposées formées à l'extrémité distale du manchon interne (12).

8. Dispositif (10) à orifice d'accès manuel selon la revendication 2, dans lequel un troisième élément de manchon est disposé à l'intérieur du manchon interne afin qu'il joue le rôle d'une soupape intermédiaire autour de la main du chirurgien entre le dispositif d'étanchéité d'entrée et le dispositif d'étanchéité de sortie.

9. Dispositif (10) à orifice d'accès manuel selon l'une quelconque des revendications précédentes, dans lequel l'élément d'ancrage comporte un élément d'anneau destiné à être monté à l'extérieur du corps du patient.

10. Dispositif (10) à orifice d'accès manuel selon l'une quelconque des revendications précédentes, dans lequel le dispositif est formé de matériau de matière plastique/polymère souple, imperméable, biocompatible médicalement et stérile.
